(19) **Europäisches Patentamt / European Patent Office / Office européen des brevets**

(11) **EP 4 752 244 A1**

(12) # EUROPEAN PATENT APPLICATION
published in accordance with Art. 153(4) EPC

(43) Date of publication:
**03.06.2026 Bulletin 2026/23**

(21) Application number: **24940894.9**

(22) Date of filing: **30.09.2024**

(51) International Patent Classification (IPC):
*C22B 23/00* (2006.01)   *C22B 3/08* (2006.01)

(86) International application number:
**PCT/CN2024/122779**

(87) International publication number:
**WO 2026/065410 (02.04.2026 Gazette 2026/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Gem Co., Ltd.**
  **Shenzhen, Guangdong 518101 (CN)**
• **PT Green Eco Nickel**
  **Jakarta Selatan, Provinsi DKI Jakarta, 12950 (ID)**
• **PT ESG New Energy Material**
  **Jakarta Selatan, Provinsi DKI Jakarta 12950 (ID)**
• **PT QMB New Energy Materials**
  **Jakarta Selatan, Provinsi DKI Jakarta, 12950 (ID)**

(72) Inventors:
• **XU, Kaihua**
  **Shenzhen, Guangdong 518100 (CN)**
• **BRODJONEGORO, Satryo Soemantri**
  **Kota Adm. Jakarta Selatan,**
  **Provinsi DKI Jakarta 12950 (ID)**
• **WANG, Yaning**
  **Kota Adm. Jakarta Selatan,**
  **Provinsi DKI Jakarta 12950 (ID)**
• **AJI, Tegar Mukti**
  **Kota Adm. Jakarta Selatan,**
  **Provinsi DKI Jakarta 12950 (ID)**

• **WANG, Yi**
  **Kota Adm. Jakarta Selatan,**
  **Provinsi DKI Jakarta 12950 (ID)**
• **YANG, Jian**
  **Kota Adm. Jakarta Selatan,**
  **Provinsi DKI Jakarta 12950 (ID)**
• **WANALDI, Rizky**
  **Kota Adm. Jakarta Selatan,**
  **Provinsi DKI Jakarta 12950 (ID)**
• **KRISTIYANTO, Evan Wahyu**
  **Kota Adm. Jakarta Selatan,**
  **Provinsi DKI Jakarta 12950 (ID)**
• **ROHMAWATI, Ulfi**
  **Kota Adm. Jakarta Selatan,**
  **Provinsi DKI Jakarta 12950 (ID)**
• **ARINDA, Shella**
  **Kota Adm. Jakarta Selatan,**
  **Provinsi DKI Jakarta 12950 (ID)**
• **JABIR, Ardi Alam**
  **Kota Adm. Jakarta Selatan,**
  **Provinsi DKI Jakarta 12950 (ID)**
• **RAHMADI, Piyan**
  **Kota Adm. Jakarta Selatan,**
  **Provinsi DKI Jakarta 12950 (ID)**

(74) Representative: **Biallo, Dario**
  **Barzanò & Zanardo S.p.A.**
  **Via Borgonuovo, 10**
  **20121 Milano (IT)**

(54) **METHOD AND PROCESS FOR ENHANCED LEACHING OF LATERITIC NICKEL ORE**

(57)   The present application provides a method and a process for strengthened leaching of laterite nickel ore. The method comprises: obtaining reaction conditions for a laterite nickel ore leaching reaction; establishing a reaction model for the laterite nickel ore leaching reaction based on the reaction conditions; wherein the reaction model comprises a liquid-layer diffusion control model, a product-layer diffusion control model and a reaction control model; determining a reaction conversion yield of the laterite nickel ore leaching reaction based on the reaction model; the applicable reaction models under different reaction conditions are established based on the reaction temperature, acid/ore ratio, and reaction period, and thereby the laterite nickel ore leaching process can be controlled accurately. The present application provides theoretical basis and guidance to the strengthened leaching of laterite nickel ore, and thusly improving the reaction rate of the laterite nickel ore leaching reaction.

EP 4 752 244 A1

| | |
|---|---|
| Obtaining reaction conditions for a laterite nickel ore leaching reaction, wherein the reaction conditions comprise a reaction temperature, an acid/ore ratio and a reaction period | S101 |

| | |
|---|---|
| Establishing a reaction model for the laterite nickel ore leaching reaction based on the reaction conditions; wherein the reaction model comprises a liquid-layer diffusion control model, a product-layer diffusion control model and a reaction control model | S102 |

| | |
|---|---|
| Determining a reaction conversion yield of the laterite nickel ore leaching reaction based on the reaction model | S103 |

| | |
|---|---|
| Determining a predicted gross profit of the laterite nickel ore leaching reaction based on the reaction conditions and the reaction conversion yield, and determining whether the predicted gross profit satisfies a target gross profit | S104 |

| | |
|---|---|
| In a case where the predicted gross profit satisfies the target gross profit, using the reaction conditions as target reaction conditions; in a case where the predicted gross profit does not satisfy the target gross profit, optimizing the reaction conditions until the predicted gross profit satisfies the target gross profit | S105 |

FIG. 1

**Description**

TECHNICAL FIELD

The present application relates to the technical field of hydrometallurgy of laterite nickel ore, and specifically relates to a method and a process for strengthened leaching of laterite nickel ore.

BACKGROUND

In the hydrometallurgical process of laterite nickel ore, the acid leaching process is one of the most important processes, by which the laterite nickel ore slurry is mixed with a strong acid solution in a high-temperature, high-pressure autoclave and subjected to acid leaching to extract nickel, cobalt, manganese and other metals, and then after the purification treatment, the MHP product is obtained. The recovery rate of nickel and cobalt metals can reach 90% or more by such process.

However, because the acid leaching process is a complex multi-phase reaction process, it is difficult to accurately anticipate the reaction process or result. In the production practice, the leaching rate of the metal needs to be continuously improved to increase the production efficiency and reduce the production cost. However, to get the optimal nickel and cobalt leaching rate needs performing a number of experiments at different conditions, or even a number of experiments are not enough to get the optimal leaching rate.

Therefore, there is an urgent need to provide a method and a process for strengthened leaching of laterite nickel ore to improve the leaching efficiency of high-pressure acid leaching of laterite nickel ore.

SUMMARY

In view of the above, it is necessary to provide a method and a process for strengthened leaching of laterite nickel ore to solve the technical problem of the prior art that the reaction in the acid leaching process of laterite nickel ore cannot be described clearly; the technical problem will result in that the optimal leaching efficiency cannot be effectively found and further lead to a low leaching efficiency.

In an aspect, in order to solve the above technical problem, the present application provides a method for strengthened leaching of laterite nickel ore, comprising:

obtaining reaction conditions for a laterite nickel ore leaching reaction; wherein the reaction conditions comprise a reaction temperature, an acid/ore ratio and a reaction period;

establishing a reaction model for the laterite nickel ore leaching reaction based on the reaction conditions; wherein the reaction model comprises a liquid-layer diffusion control model, a product-layer diffusion control model and a reaction control model;

determining a reaction conversion yield of the laterite nickel ore leaching reaction based on the reaction model;

determining a predicted gross profit of the laterite nickel ore leaching reaction based on the reaction conditions and the reaction conversion yield, and determining whether the predicted gross profit satisfies a target gross profit; and

in a case where the predicted gross profit satisfies the target gross profit, using the reaction conditions as target reaction conditions; in a case where the predicted gross profit does not satisfy the target gross profit, optimizing the reaction conditions until the predicted gross profit satisfies the target gross profit;

wherein a method of optimizing the reaction conditions comprises at least one of increasing the reaction temperature, increasing the acid/ore ratio and extending the reaction period.

In an optional embodiment, the process of establishing the reaction model for the laterite nickel ore leaching reaction based on the reaction temperature, the acid/ore ratio and the reaction period comprises:

determining whether the reaction temperature is greater than a temperature threshold;

in a case where the reaction temperature is less than or equal to the temperature threshold, establishing the reaction model for the laterite nickel ore leaching reaction based on a first selection strategy, the acid/ore ratio and the reaction period; and

in a case where the reaction temperature is greater than the temperature threshold, establishing the reaction model for the laterite nickel ore leaching reaction based on a second selection strategy, the acid/ore ratio and the reaction period.

In an optional embodiment, the process of establishing the reaction model for the laterite nickel ore leaching reaction based on the first selection strategy, the acid/ore ratio and the reaction period comprises:

determining whether the acid/ore ratio is greater than a first acid/ore ratio threshold;

in a case where the acid/ore ratio is less than or equal to the first acid/ore ratio threshold, using the reaction control model as the reaction model;

in a case where the acid/ore ratio is greater than the first acid/ore ratio threshold, determining whether the reaction period is less than a first reaction period threshold;

in a case where the reaction period is less than the first reaction period threshold, using the liquid-layer diffusion control model as the reaction model; and

in a case where the reaction period is greater than or equal to the first reaction period threshold, using the product-layer diffusion control model as the reaction model.

In an optional embodiment, the process of establishing the reaction model for the laterite nickel ore leaching reaction based on the second selection strategy, the acid/ore ratio and the reaction period comprises:

determining whether the acid/ore ratio is greater than a second acid/ore ratio threshold; wherein the second acid/ore ratio threshold is less than the first acid/ore ratio threshold;

in a case where the acid/ore ratio is less than or equal to the second acid/ore ratio threshold, using the liquid-layer diffusion control model as the reaction model;

in a case where the acid/ore ratio is greater than the second acid/ore ratio threshold, determining whether the reaction period is less than a second reaction period threshold; wherein the second reaction period threshold is less than the first reaction period threshold;

in a case where the reaction period is less than the second reaction period threshold, using the liquid-layer diffusion control model as the reaction model; and

in a case where the reaction period is greater than or equal to the second reaction period threshold, using the product-layer diffusion control model as the reaction model.

In an optional embodiment, the temperature threshold is 200°C, the first acid/ore ratio is 0.35, the second acid/ore ratio is 0.3, the first reaction period threshold is 60 minutes, and the second reaction period threshold is 50 minutes.
In an optional embodiment, the liquid-layer diffusion control model is:

$$r_B = \frac{3bk_cC_A\varepsilon_s}{R_0}(1 - \alpha X)$$

wherein $r_B$ represents a leaching rate of a reactant; $b$ represents a stoichiometric coefficient of the reactant; $k_c$ represents a mass transfer coefficient of an acid solution; $C_A$ represents a molar concentration of the acid solution; $\varepsilon_S$ represents a volume fraction of the reactant; $R_0$ represents an initial particle size of ore; $\alpha$ represents a stoichiometric ratio of the acid solution to the reactant; and X represents the reaction conversion yield.
In an optional embodiment, the product-layer diffusion control model is:

$$r_B = \frac{3\varepsilon_s bC_A D_e}{R_0^2}\frac{(1-X)^{1/3}}{1-(1-X)^{1/3}}$$

wherein $r_B$ represents a leaching rate of a reactant; $b$ represents a stoichiometric coefficient of the reactant; $C_A$ represents a molar concentration of an acid solution; $\varepsilon_S$ represents a volume fraction of the reactant; $R_0$ represents an initial particle size of ore; $X$ represents the reaction conversion yield; and $D_e$ represents an effective diffusion coefficient of the reactant.

In an optional embodiment, the reaction control model is:

$$r_B = \frac{3bk_R C_A \varepsilon_s(1 - \alpha X)}{R_0}(1 - X)^{2/3}$$

wherein $r_B$ represents a leaching rate of a reactant; $b$ represents a stoichiometric coefficient of the reactant; $C_A$ represents a molar concentration of an acid solution; $\varepsilon_S$ represents a volume fraction of the reactant; $R_0$ represents an initial particle size of ore; $X$ represents the reaction conversion yield; and $k_R$ represents a reaction rate constant.

In an optional embodiment, the method further comprises:

obtaining a molar density of a reactant, a cross-sectional area of a reactor, a solid feed density, a solid feed rate and a length of the reactor in the laterite nickel ore leaching reaction; and

determining a leaching amount of the laterite nickel ore leaching reaction based on the reaction conversion yield, the molar density of the reactant, the cross-sectional area of the reactor, the solid feed density, the solid feed rate and the length of the reactor.

In another aspect, the present application also provides a process for strengthened leaching of laterite nickel ore, which is realized based on a method for strengthened leaching of laterite nickel ore;
wherein the method for strengthened leaching of laterite nickel ore is the method for strengthened leaching of laterite nickel ore according to any one of the optional embodiments.

The beneficial effects of the present application are as follows: the method for strengthened leaching of laterite nickel ore provided by the present application describes the reaction mechanism of the laterite nickel ore leaching reaction by dividing the reaction model of the laterite nickel ore leaching reaction into the liquid-layer diffusion control model, the product-layer diffusion control model, and the reaction control model. Moreover, the applicable reaction models under different reaction conditions are established based on the reaction temperature, acid/ore ratio, and reaction period, and thereby the laterite nickel ore leaching process can be controlled accurately, the strengthened leaching of laterite nickel ore can be provided with theoretical basis and guidance, and thusly the reaction rate of the laterite nickel ore leaching reaction can be improved. Furthermore, the predicted gross profit of the laterite nickel ore leaching reaction is determined based on the reaction conditions and reaction conversion yield, and the reaction conditions are adjusted based on the predicted gross profit to reach the target reaction conditions that meet the target gross profit. That is, the optimal reaction conditions corresponding to the target gross profit and the corresponding optimal reaction conversion yield are found to strengthen the leaching rate of high-pressure acid leaching of laterite nickel ore.

BRIEF DESCRIPTION OF DRAWINGS

In order to more clearly illustrate the technical solutions in the embodiments of the present application, the accompanying drawings to be used in the description of the embodiments will be briefly introduced below. It is obvious that the accompanying drawings in the following description are only some of the embodiments of the present application, and other drawings can be obtained based on these accompanying drawings by those skilled in the art without creative efforts.

FIG. 1 shows a schematic flow diagram of an embodiment of the method for strengthened leaching of laterite nickel ore provided by the present application;

FIG. 2 shows a schematic flow diagram of an embodiment of S102 in FIG. 1 of the present application;

FIG. 3 shows a schematic flow diagram of an embodiment of S202 in FIG. 2 of the present application;

FIG. 4 shows a schematic flow diagram of an embodiment of S203 of FIG. 2 of the present application;

FIG. 5 shows a schematic diagram of a specific embodiment of establishing the reaction model provided by the present application;

FIG. 6 shows a schematic flow diagram of an embodiment of determining a leaching amount for the laterite nickel ore leaching method provided by the present application.

DETAILED DESCRIPTION

The technical solutions in the embodiments of the present application will be clearly and completely described below in conjunction with the accompanying drawings in the embodiments of the present application. Obviously, the described embodiments are only a part of the embodiments of the present application, and not all of the embodiments. Based on the embodiments in the present application, all other embodiments obtained by those skilled in the art without creative efforts fall within the scope of protection of the present application.

It should be understood that the schematic diagrams are not drawn to physical scale. The flow diagrams used in the present application show operations in accordance with some embodiments of the present application. It should be understood that the operations of the flow diagrams may be implemented out of order, and steps that have no logical contextual order may be implemented in reverse order or simultaneously. In addition, those skilled in the art may add one or more operations to the flow diagram or may remove one or more operations from the flow diagram under the guidance of the contents of the present application. Some of the block diagrams shown in the accompanying drawings are functional entities that do not necessarily correspond to physical or logical individuals. These functional entities may be implemented in software, or in one or more hardware modules or integrated circuits, or in different networks and/or processor systems and/or microcontroller systems.

The expression "embodiment" referred to herein implies that the particular features, structures, or characteristics described via the embodiment may be included in at least one embodiment of the present application. The expression appearing at various positions in the specification does not necessarily refer to the same embodiment or an independent or alternative embodiment incompatible with other embodiments. It is understood by those skilled in the art, both explicitly and implicitly, that the embodiments described herein may be combined with other embodiments.

The present application provides a method and a process for strengthened leaching of laterite nickel ore, each of which is described below.

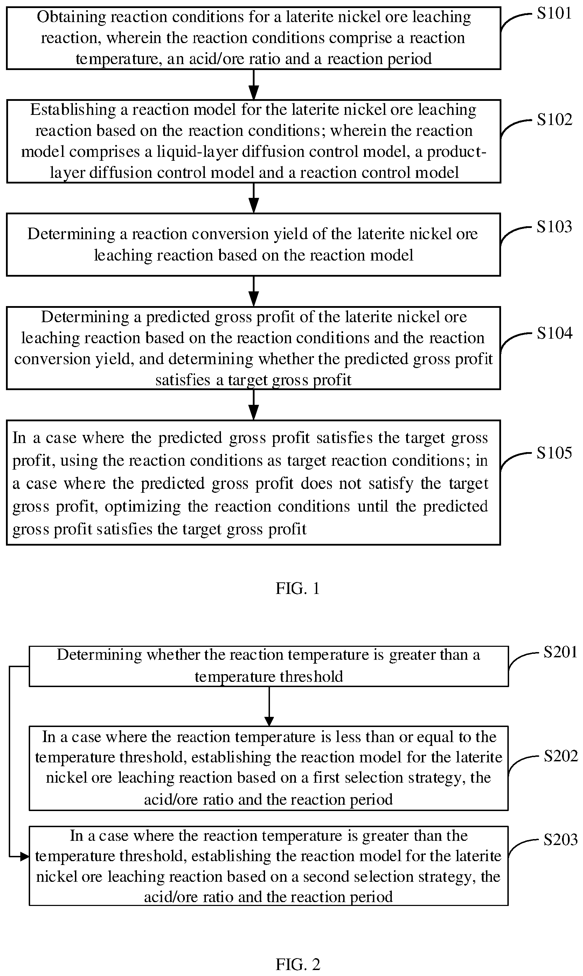

FIG. 1 shows a schematic flow diagram of an embodiment of the method for strengthened leaching of laterite nickel ore provided by the present application. As shown in FIG. 1, the method for strengthened leaching of laterite nickel ore comprises:

S101. obtaining reaction conditions for a laterite nickel ore leaching reaction, wherein the reaction conditions comprise a reaction temperature, an acid/ore ratio and a reaction period;

S102. establishing a reaction model for the laterite nickel ore leaching reaction based on the reaction conditions; wherein the reaction model comprises a liquid-layer diffusion control model, a product-layer diffusion control model and a reaction control model;

S103. determining a reaction conversion yield of the laterite nickel ore leaching reaction based on the reaction model;

S104. determining a predicted gross profit of the laterite nickel ore leaching reaction based on the reaction conditions and the reaction conversion yield, and determining whether the predicted gross profit satisfies a target gross profit;

S105. in a case where the predicted gross profit satisfies the target gross profit, using the reaction conditions as target reaction conditions; in a case where the predicted gross profit does not satisfy the target gross profit, optimizing the reaction conditions until the predicted gross profit satisfies the target gross profit;

wherein a method of optimizing the reaction conditions comprises at least one of increasing the reaction temperature, increasing the acid/ore ratio and extending the reaction period.

It should be noted that: in some application practices, the reaction conditions also include a stirring speed; the stirring speed is not considered when establishing the reaction model, but when optimizing the reaction conditions, the optimization method can also include increasing the stirring speed to increase the diversity of the optimization method of the reaction conditions.

The reaction equation for the laterite nickel ore leaching reaction is:

$$A_{(l)} + bB_{(s)} \rightarrow cC$$

$$N_B = N_{B0}(1 - X)$$

wherein $A_{(l)}$ represents an acid solution; $B_{(s)}$ represents a reactant, such as nickel oxide, cobalt oxide, etc.; C represents an extract; b represents a stoichiometric coefficient of the reactant; c represents a stoichiometric coefficient of the extract; $N_B$ represents a real-time molar mass of the reactant in the reactor, mol; $N_{B0}$ represents an initial molar mass of the reactant in the reactor, mol; and X represents the reaction conversion yield,%.

It should be understood that since the reactant includes nickel and cobalt, the reaction conversion yield in step S103 includes the reaction conversion yield of nickel and the reaction conversion yield of cobalt.

Specifically,

$$\text{Gross profit} = \sum \text{Revenue} - \sum \text{Cost}$$

In the above formula, the revenue= raw ore processing amount * (Ni content in raw ore * Reaction conversion yield of Ni * Price of Ni+ Co content in raw ore * Reaction conversion yield of Co * Price of Co).

In the above formula, the cost= Steam usage * Steam price+ Sulfuric acid usage * Sulfuric acid price+ Electricity price * Electricity consumption. It should be understood that the steam usage is related to the reaction temperature in the reaction conditions, the sulfuric acid usage is related to the acid/ore ratio in the reaction conditions, and the electricity consumption is related to the reaction period in the reaction conditions.

In other words, step S104 is specifically: determining the cost based on the reaction conditions, determining the revenue based on the reaction conversion yield, and determining the predicted gross profit based on the cost and the revenue.

Compared with the prior art, the method for strengthened leaching of laterite nickel ore provided by the present application describes the reaction mechanism of the laterite nickel ore leaching reaction by dividing the reaction model of the laterite nickel ore leaching reaction into the liquid-layer diffusion control model, the product-layer diffusion control model, and the reaction control model. Moreover, the applicable reaction models under different reaction conditions are established based on the reaction temperature, acid/ore ratio, and reaction period, and thereby the laterite nickel ore leaching process can be controlled accurately, the strengthened leaching of laterite nickel ore can be provided with theoretical basis and guidance, and thusly the reaction rate of the laterite nickel ore leaching reaction can be improved. Furthermore, the predicted gross profit of the laterite nickel ore leaching reaction is determined based on the reaction conditions and reaction conversion yield, and the reaction conditions are adjusted based on the predicted gross profit to reach the target reaction conditions that meet the target gross profit. That is, the optimal reaction conditions corresponding to the target gross profit and the corresponding optimal reaction conversion yield are found to strengthen the leaching rate of high-pressure acid leaching of laterite nickel ore.

To improve the orderliness of the reaction model determination, in some embodiments of the present application, as shown in FIG. 2, step S102 comprises:

S201. determining whether the reaction temperature is greater than a temperature threshold;

S202. in a case where the reaction temperature is less than or equal to the temperature threshold, establishing the reaction model for the laterite nickel ore leaching reaction based on a first selection strategy, the acid/ore ratio and the reaction period; and

S203. in a case where the reaction temperature is greater than the temperature threshold, establishing the reaction model for the laterite nickel ore leaching reaction based on a second selection strategy, the acid/ore ratio and the reaction period.

In the embodiment of the present application, considering that the reaction temperature is a routine term in the reaction conditions, the relationship between the reaction temperature and the temperature threshold is firstly used to divide the process of establishing the reaction model into the first selection strategy and the second selection strategy, i.e., the reaction temperature is taken as the primary condition for the determination of the reaction model, which improves the orderliness and efficiency of the determination of the reaction model.

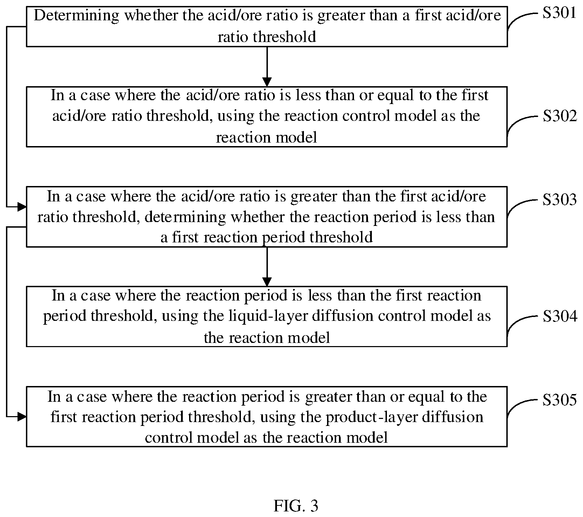

In a specific embodiment of the present application, as shown in FIG. 3, step S202 comprises:

S301. determining whether the acid/ore ratio is greater than a first acid/ore ratio threshold;

S302. in a case where the acid/ore ratio is less than or equal to the first acid/ore ratio threshold, using the reaction control model as the reaction model;

S303. in a case where the acid/ore ratio is greater than the first acid/ore ratio threshold, determining whether the reaction period is less than a first reaction period threshold;

S304. in a case where the reaction period is less than the first reaction period threshold, using the liquid-layer diffusion control model as the reaction model; and

S305. in a case where the reaction period is greater than or equal to the first reaction period threshold, using the product-layer diffusion control model as the reaction model.

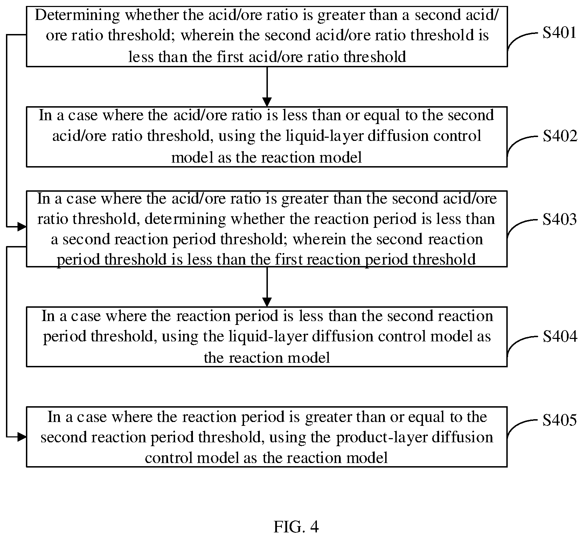

In a specific embodiment of the present application, as shown in FIG. 4, step S203 comprises:

S401. determining whether the acid/ore ratio is greater than a second acid/ore ratio threshold; wherein the second acid/ore ratio threshold is less than the first acid/ore ratio threshold;

S402. in a case where the acid/ore ratio is less than or equal to the second acid/ore ratio threshold, using the liquid-layer diffusion control model as the reaction model;

S403. in a case where the acid/ore ratio is greater than the second acid/ore ratio threshold, determining whether the reaction period is less than a second reaction period threshold; wherein the second reaction period threshold is less than the first reaction period threshold;

S404. in a case where the reaction period is less than the second reaction period threshold, using the liquid-layer diffusion control model as the reaction model; and

S405. in a case where the reaction period is greater than or equal to the second reaction period threshold, using the product-layer diffusion control model as the reaction model.

In the embodiment of the present application, the acid/ore ratio and the reaction period are used to accurately select the three reaction models: the liquid-layer diffusion control model, the product-layer diffusion control model and the reaction control model, and thus the accuracy of the finally determined reaction conversion yield is improved, providing the theoretical basis for the strengthened leaching reaction of laterite nickel ore.

It should be understood that the temperature threshold, the first acid/ore ratio threshold, the second acid/ore ratio threshold, the first reaction period threshold, and the second reaction period threshold may be determined or adjusted according to the specific application practice.

In a specific embodiment of the present application, the temperature threshold is 200°C, the first acid/ore ratio is 0.35, the second acid/ore ratio is 0.3, the first reaction period threshold is 60 minutes, and the second reaction period threshold is 50 minutes.

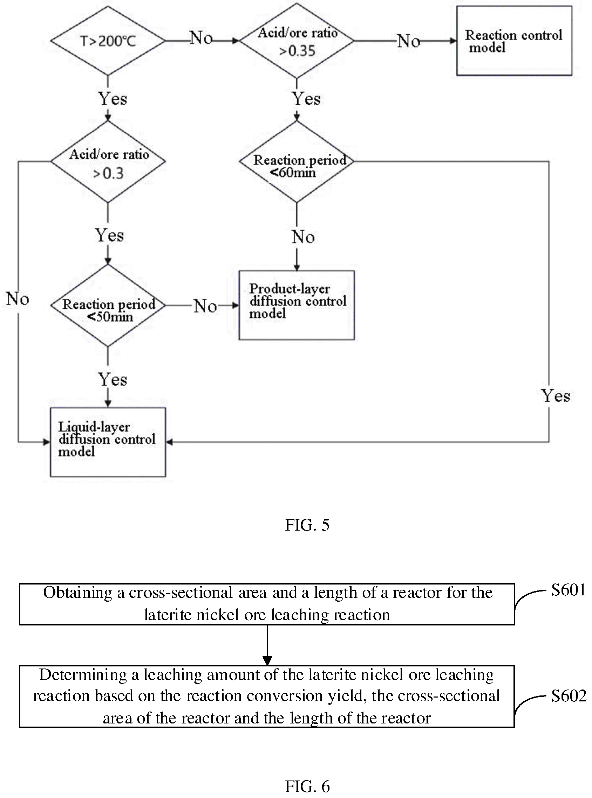

In a specific embodiment of the present application, as shown in FIG. 5, the specific process of establishing the reaction model is as follows: it is determined whether the reaction temperature is greater than 200°C; in a case where the reaction temperature is less than or equal to 200°C, it is determined whether the acid/ore ratio is greater than 0.35; in a case where the acid/ore ratio is less than or equal to 0.35, the reaction control model is used as the reaction model; in a case where the acid/ore ratio is greater than 0.35, it is determined whether the reaction period is less than 60 minutes; in a case where the reaction period is less than 60 minutes, the liquid-layer diffusion control model is used as the reaction model; and in a case where the reaction period is greater than or equal to 60 minutes, the product-layer diffusion control model is used as the reaction model. In a case where the reaction temperature is greater than 200°C, it is determined whether the acid/ore ratio is greater than 0.3; in a case where the acid/ore ratio is less than or equal to 0.3, the liquid-layer diffusion control model is used as the reaction model; in a case where the acid/ore ratio is greater than 0.3, it is determined whether the reaction period is less than 50 minutes; in a case where the reaction period is less than 50 minutes, the liquid-layer diffusion control model is used as the reaction model; and in a case where the reaction period is greater than or equal to 50 minutes, the product-layer diffusion control model is used as the reaction model.

In a specific embodiment of the present application, the liquid-layer diffusion control model is:

$$r_B = \frac{3bk_c C_A \varepsilon_s}{R_0}(1 - \alpha X)$$

wherein $r_B$ represents a leaching rate of a reactant, mol/m³ • s; $b$ represents a stoichiometric coefficient of the reactant; $k_c$ represents a mass transfer coefficient of an acid solution; $C_A$ represents a molar concentration of the acid solution, mol/m³; $\varepsilon_S$ represents a volume fraction of the reactant; $R_0$ represents an initial particle size of ore, m; $\alpha$ represents a stoichiometric ratio of the acid solution to the reactant; and $X$ represents the reaction conversion yield.

The specific derivation process is as follows:

$$R_B = -\frac{1}{s_p}\frac{dN_B}{dt} = bR_A = bk_cC_A(1 - \alpha X)$$

$$S_p = 4\pi R_0^2$$

$$r_B = R_B S_p n_p$$

wherein $s_p$ represents an available reaction area, m²; $R_A$ represents a leaching rate per unit area of an acid solution, mol/m²•s; $R_B$ represents a leaching rate per unit area of a reactant, mol/m²•s; $R_0$ represents an initial particle size of ore, m; and $n_p$ represents the number of reactive particles per unit volume (m⁻³),

$$n_p = \frac{3\varepsilon_s}{4\pi R_0^3}$$

then

$$r_B = \frac{3bk_cC_A\,\varepsilon_s}{R_0}(1 - \alpha X)$$

In a specific embodiment of the present application, the product-layer diffusion control model is:

$$r_B = \frac{3\varepsilon_s bC_A D_e}{R_0^2}\frac{(1 - X)^{1/3}}{1 - (1 - X)^{1/3}}$$

wherein $r_B$ represents a leaching rate of a reactant, mol/m³•s; $b$ represents a stoichiometric coefficient of the reactant; $C_A$ represents a molar concentration of an acid solution, mol/m³; $\varepsilon_S$ represents a volume fraction of the reactant; $R_0$ represents an initial particle size of ore, m; $X$ represents the reaction conversion yield; and $D_e$ represents an effective diffusion coefficient of the reactant.

The specific derivation process is as follows:

$$R_B = -\frac{1}{s_p}\frac{dN_B}{dt} = bR_A = bJ_A|_{R=r}$$

$$S_p = 4\pi R_0^2$$

The diffusion flux obeys Fick's law. In order to further derive the equation, it is necessary to acquire the concentration distribution of component A in the solid product layer. Since no chemical reaction occurs in the product layer, the mass transfer rate through the solid is constant:

$$\frac{d}{dr}(4\,\pi\,r^2 J_A) = \frac{d}{dr}(4\,\pi\,r^2 D_e \frac{dC}{dr}) = 0$$

The boundary conditions for the above ordinary differential equation are:

$$C(R) = 0$$

$$C(R_0) = C_A$$

Thus, the concentration distribution in the solid layer is

$$C = \frac{\frac{1}{R} - \frac{1}{r}}{\frac{1}{R} - \frac{1}{R_0}} C_A$$

$$\left.\frac{dC}{dr}\right|_{r=R} = \frac{\frac{C_A}{R^2}}{\frac{1}{R} - \frac{1}{R_0}}$$

$$n_p = \frac{3\varepsilon_s}{4\pi R_0^3}$$

$$r_B = R_B S_P n_p = 4\pi R_0^2 b D_e \frac{dC}{dr} * \frac{3\varepsilon_s}{4\pi R_0^3} = \frac{3\varepsilon_s b C_A D_e}{R_0^2} \frac{(1-X)^{1/3}}{1-(1-X)^{1/3}}$$

wherein C represents the concentration difference between the reactant and the acid solution.

In a specific embodiment of the present application, the reaction control model is:

$$r_B = \frac{3 b k_R C_A \,\varepsilon_s (1 - a X)}{R_0} (1-X)^{2/3}$$

wherein $r_B$ represents a leaching rate of a reactant, mol/m³•s; $b$ represents a stoichiometric coefficient of the reactant; $C_A$ represents a molar concentration of an acid solution, mol/m³•s; $\varepsilon_S$ represents a volume fraction of the reactant; $R_0$ represents an initial particle size of ore, m; $X$ represents the reaction conversion yield; and $k_R$ represents a reaction rate constant.

The specific derivation process is as follows:

$$R_B = -\frac{1}{S_p}\frac{dN_B}{dt} = b R_A = b k_R C_A$$

$$S_p = 4\pi R^2$$

wherein $k_R$ is the reaction rate constant, 1/s,

$$r_B = \frac{3bk_R C_A \, \varepsilon_s (1 - \alpha X)}{R_0}(1 - X)^{2/3}$$

Since the practical engineering application is more interested in the leaching amount of the laterite nickel ore leaching reaction, in some embodiments of the present application, as shown in FIG. 6, the method for strengthened leaching of laterite nickel ore further comprises:

S601. obtaining a cross-sectional area of a reactor for the laterite nickel ore leaching reaction and a length of the reactor; and
S602. determining a leaching amount of the laterite nickel ore leaching reaction based on the reaction conversion yield, the cross-sectional area of the reactor, and the length of the reactor.

In the embodiments of the present application, the leaching amount of the laterite nickel ore leaching reaction is determined based on the reaction conversion yield, further providing guidance for the practical industrial application, providing optimization direction for laterite nickel ore leaching, and further improving the leaching amount of the laterite nickel ore leaching reaction.

It should be understood that the leaching amount is the integral of the reaction conversion yield per unit length along the length of the reactor, and the reaction conversion yield per unit length is:

$$\frac{dX}{dz} = \frac{3\varepsilon_s \rho_p S}{4\pi R_0^3 \rho_B F} R_B S_P$$

wherein $z$ represents a length of a reactor; $S$ represents a cross-sectional area of the reactor; $\varepsilon_s$ represents a volume fraction of a reactant; $\rho_B$ represents a molar density of the reactant; $\rho_p$ represents a solid feed density; $R_B$ represents a leaching rate per unit area of the reactant; and $F$ represents a solid feed rate.

When the reaction model is the liquid-layer diffusion control model:

$$\frac{dX}{dz} = \frac{3\varepsilon_s \rho_p S}{4\pi R_0^3 \rho_B F} R_B S_P = \frac{3bk_c C_A \varepsilon_s \rho_p S}{R_0 \rho_B F}(1 - \alpha X)$$

Since X=0 when z=0, then:

$$-\ln(1 - \alpha X) = \frac{3bk_c C_A \varepsilon_s \rho_p S}{R_0 \rho_B F} \alpha z$$

When the reaction model is the product-layer diffusion control model:

$$\frac{dX}{dz} = \frac{3\varepsilon_s b C_A D_e \rho_p S (1 - \alpha X)}{R_0^2 \rho_B F} \frac{(1 - X)^{1/3}}{1 - (1 - X)^{1/3}}$$

$$2\int_0^X \frac{[1 - (1 - X)^{1/3}]}{(1 - \alpha X)(1 - X)^{1/3}} dX = \frac{6\varepsilon_s b C_A D_e \rho_p S}{\rho_B R_0^2} z$$

When the reaction model is the reaction control model:

$$\frac{1}{3} \int_0^X \frac{dX}{(1-\alpha X)(1-X)^{2/3}} = \frac{3bk_R C_A \varepsilon_s}{\rho_B R_0}$$

In summary, the method for strengthened leaching of laterite nickel ore provided by the embodiment of the present application describes the different stages of the leaching process based on the liquid-layer diffusion control model, the product-layer diffusion control model, and the reaction control model, and the different control models can be switched through the three parameters of the reaction temperature, the acid/ore ratio, and the reaction period, thereby the mechanism of the leaching process of laterite nickel ore can be described accurately and in turn the accuracy of the determined reaction conversion yield can be improved. Moreover, in the embodiment of the present application, provided that the reaction conversion yield has been determined, the leaching amount is determined based on the reaction conversion yield, providing direction for the optimization of leaching amount, and improving the rationality of the laterite nickel ore leaching process.

In another aspect, an embodiment of the present application provides a process for strengthened leaching of laterite nickel ore, which is realized based on a method for strengthened leaching of laterite nickel ore;
wherein the method for strengthened leaching of laterite nickel ore is the method for strengthened leaching of laterite nickel ore according to any of the above embodiments.

It is understood by those skilled in the art that all or part of the process for realizing the method of the above embodiments may be accomplished by instructing the relevant hardware (e.g., a processor, a controller, etc.) by means of a computer program; the computer program may be stored in a computer-readable storage medium. The computer-readable storage medium is a magnetic disk, a CD-ROM, a read only memory or a random access memory, etc.

The above provides a detailed description of a method and a process for strengthened leaching of laterite nickel ore of the present application. The principles and implementations of the present application are illustrated in terms of specific examples. The above illustrations of the embodiments are only used for a better understanding of the method and core ideas of the present application; at the same time, for those skilled in the art, there will be changes in the specific embodiments and application scope based on the ideas of the present application. In conclusion, the content of this specification should not be construed as the limitation of the present application.

**Claims**

1. A method for strengthened leaching of laterite nickel ore, comprising:

    obtaining reaction conditions for a laterite nickel ore leaching reaction; wherein the reaction conditions comprise a reaction temperature, an acid/ore ratio and a reaction period;
    establishing a reaction model for the laterite nickel ore leaching reaction based on the reaction conditions; wherein the reaction model comprises a liquid-layer diffusion control model, a product-layer diffusion control model and a reaction control model;
    determining a reaction conversion yield of the laterite nickel ore leaching reaction based on the reaction model;
    determining a predicted gross profit of the laterite nickel ore leaching reaction based on the reaction conditions and the reaction conversion yield, and determining whether the predicted gross profit satisfies a target gross profit; and
    in a case where the predicted gross profit satisfies the target gross profit, using the reaction conditions as target reaction conditions; in a case where the predicted gross profit does not satisfy the target gross profit, optimizing the reaction conditions until the predicted gross profit satisfies the target gross profit;
    wherein a method of optimizing the reaction conditions comprises at least one of increasing the reaction temperature, increasing the acid/ore ratio and extending the reaction period.

2. The method for strengthened leaching of laterite nickel ore according to claim 1, wherein the process of establishing the reaction model for the laterite nickel ore leaching reaction based on the reaction temperature, the acid/ore ratio and the reaction period comprises:

    determining whether the reaction temperature is greater than a temperature threshold;
    in a case where the reaction temperature is less than or equal to the temperature threshold, establishing the reaction model for the laterite nickel ore leaching reaction based on a first selection strategy, the acid/ore ratio and the reaction period; and

in a case where the reaction temperature is greater than the temperature threshold, establishing the reaction model for the laterite nickel ore leaching reaction based on a second selection strategy, the acid/ore ratio and the reaction period.

3. The method for strengthened leaching of laterite nickel ore according to claim 2, wherein the process of establishing the reaction model for the laterite nickel ore leaching reaction based on the first selection strategy, the acid/ore ratio and the reaction period comprises:

determining whether the acid/ore ratio is greater than a first acid/ore ratio threshold;
in a case where the acid/ore ratio is less than or equal to the first acid/ore ratio threshold, using the reaction control model as the reaction model;
in a case where the acid/ore ratio is greater than the first acid/ore ratio threshold, determining whether the reaction period is less than a first reaction period threshold;
in a case where the reaction period is less than the first reaction period threshold, using the liquid-layer diffusion control model as the reaction model; and
in a case where the reaction period is greater than or equal to the first reaction period threshold, using the product-layer diffusion control model as the reaction model.

4. The method for strengthened leaching of laterite nickel ore according to claim 3, wherein the process of establishing the reaction model for the laterite nickel ore leaching reaction based on the second selection strategy, the acid/ore ratio and the reaction period comprises:

determining whether the acid/ore ratio is greater than a second acid/ore ratio threshold; wherein the second acid/ore ratio threshold is less than the first acid/ore ratio threshold;
in a case where the acid/ore ratio is less than or equal to the second acid/ore ratio threshold, using the liquid-layer diffusion control model as the reaction model;
in a case where the acid/ore ratio is greater than the second acid/ore ratio threshold, determining whether the reaction period is less than a second reaction period threshold; wherein the second reaction period threshold is less than the first reaction period threshold;
in a case where the reaction period is less than the second reaction period threshold, using the liquid-layer diffusion control model as the reaction model; and
in a case where the reaction period is greater than or equal to the second reaction period threshold, using the product-layer diffusion control model as the reaction model.

5. The method for strengthened leaching of laterite nickel ore according to claim 4, wherein the temperature threshold is 200°C, the first acid/ore ratio is 0.35, the second acid/ore ratio is 0.3, the first reaction period threshold is 60 minutes, and the second reaction period threshold is 50 minutes.

6. The method for strengthened leaching of laterite nickel ore according to claim 1, wherein the liquid-layer diffusion control model is:

$$r_B = \frac{3 b k_c C_A \varepsilon_s}{R_0}(1 - \alpha X)$$

wherein $r_B$ represents a leaching rate of a reactant; $b$ represents a stoichiometric coefficient of the reactant; $k_c$ represents a mass transfer coefficient of an acid solution; $C_A$ represents a molar concentration of the acid solution; $\varepsilon_S$ represents a volume fraction of the reactant; $R_0$ represents an initial particle size of ore; $\alpha$ represents a stoichiometric ratio of the acid solution to the reactant; and X represents the reaction conversion yield.

7. The method for strengthened leaching of laterite nickel ore according to claim 1, wherein the product-layer diffusion control model is:

$$r_B = \frac{3 \varepsilon_s b C_A D_e}{R_0^2} \frac{(1 - X)^{1/3}}{1 - (1 - X)^{1/3}}$$

wherein $r_B$ represents a leaching rate of a reactant; $b$ represents a stoichiometric coefficient of the reactant; $C_A$ represents a molar concentration of an acid solution; $\varepsilon_S$ represents a volume fraction of the reactant; $R_0$ represents an initial particle size of ore; $X$ represents the reaction conversion yield; and $D_e$ represents an effective diffusion coefficient of the reactant.

8.  The method for strengthened leaching of laterite nickel ore according to claim 1, wherein the reaction control model is:

$$r_B = \frac{3bk_R C_A \varepsilon_s (1 - \alpha X)}{R_0}(1 - X)^{2/3}$$

wherein $r_B$ represents a leaching rate of a reactant; $b$ represents a stoichiometric coefficient of the reactant; $C_A$ represents a molar concentration of an acid solution; $\varepsilon_S$ represents a volume fraction of the reactant; $R_0$ represents an initial particle size of ore; $X$ represents the reaction conversion yield; and $k_R$ represents a reaction rate constant.

9.  The method for strengthened leaching of laterite nickel ore according to claim 1, wherein the method further comprises:

    obtaining a cross-sectional area and a length of a reactor for the laterite nickel ore leaching reaction; and
    determining a leaching amount of the laterite nickel ore leaching reaction based on the reaction conversion yield, the cross-sectional area of the reactor and the length of the reactor.

10. A process for strengthened leaching of laterite nickel ore, which is realized based on a method for strengthened leaching of laterite nickel ore;
    wherein the method for strengthened leaching of laterite nickel ore is the method for strengthened leaching of laterite nickel ore according to any one of claims 1-9.

| Obtaining reaction conditions for a laterite nickel ore leaching reaction, wherein the reaction conditions comprise a reaction temperature, an acid/ore ratio and a reaction period | S101 |

| Establishing a reaction model for the laterite nickel ore leaching reaction based on the reaction conditions; wherein the reaction model comprises a liquid-layer diffusion control model, a product-layer diffusion control model and a reaction control model | S102 |

| Determining a reaction conversion yield of the laterite nickel ore leaching reaction based on the reaction model | S103 |

| Determining a predicted gross profit of the laterite nickel ore leaching reaction based on the reaction conditions and the reaction conversion yield, and determining whether the predicted gross profit satisfies a target gross profit | S104 |

| In a case where the predicted gross profit satisfies the target gross profit, using the reaction conditions as target reaction conditions; in a case where the predicted gross profit does not satisfy the target gross profit, optimizing the reaction conditions until the predicted gross profit satisfies the target gross profit | S105 |

FIG. 1

| Determining whether the reaction temperature is greater than a temperature threshold | S201 |

| In a case where the reaction temperature is less than or equal to the temperature threshold, establishing the reaction model for the laterite nickel ore leaching reaction based on a first selection strategy, the acid/ore ratio and the reaction period | S202 |

| In a case where the reaction temperature is greater than the temperature threshold, establishing the reaction model for the laterite nickel ore leaching reaction based on a second selection strategy, the acid/ore ratio and the reaction period | S203 |

FIG. 2

Determining whether the acid/ore ratio is greater than a first acid/ore ratio threshold ⌐ S301

In a case where the acid/ore ratio is less than or equal to the first acid/ore ratio threshold, using the reaction control model as the reaction model ⌐ S302

In a case where the acid/ore ratio is greater than the first acid/ore ratio threshold, determining whether the reaction period is less than a first reaction period threshold ⌐ S303

In a case where the reaction period is less than the first reaction period threshold, using the liquid-layer diffusion control model as the reaction model ⌐ S304

In a case where the reaction period is greater than or equal to the first reaction period threshold, using the product-layer diffusion control model as the reaction model ⌐ S305

FIG. 3

Determining whether the acid/ore ratio is greater than a second acid/
ore ratio threshold; wherein the second acid/ore ratio threshold is
less than the first acid/ore ratio threshold — S401

In a case where the acid/ore ratio is less than or equal to the second
acid/ore ratio threshold, using the liquid-layer diffusion control
model as the reaction model — S402

In a case where the acid/ore ratio is greater than the second acid/ore
ratio threshold, determining whether the reaction period is less than
a second reaction period threshold; wherein the second reaction
period threshold is less than the first reaction period threshold — S403

In a case where the reaction period is less than the second reaction
period threshold, using the liquid-layer diffusion control model as
the reaction model — S404

In a case where the reaction period is greater than or equal to the
second reaction period threshold, using the product-layer diffusion
control model as the reaction model — S405

FIG. 4

FIG. 5

| Obtaining a cross-sectional area and a length of a reactor for the laterite nickel ore leaching reaction | S601 |

| Determining a leaching amount of the laterite nickel ore leaching reaction based on the reaction conversion yield, the cross-sectional area of the reactor and the length of the reactor | S602 |

FIG. 6

# INTERNATIONAL SEARCH REPORT

| International application No. |
| --- |
| **PCT/CN2024/122779** |

**A. CLASSIFICATION OF SUBJECT MATTER**

C22B23/00(2006.01)i; C22B3/08(2006.01)i

According to International Patent Classification (IPC) or to both national classification and IPC

**B. FIELDS SEARCHED**

Minimum documentation searched (classification system followed by classification symbols)

IPC: C22B

Documentation searched other than minimum documentation to the extent that such documents are included in the fields searched

Electronic data base consulted during the international search (name of data base and, where practicable, search terms used)

CNTXT, CNKI, DWPI, WPABS, WPABSC: 红土镍矿, 浸出, 反应温度, 酸矿比, 反应时间, 反应模型, 液体层扩散控制模型, 产物层扩散控制模型, 反应控制模型, 转化率, 毛利, 优化, 阀值, 选择策略, 反应釜, laterite-nickel ore, leaching, reaction temperature, acid-ore ratio, reaction time, reaction model, liquid layer diffusion control model, products layer diffusion control model, reaction control model, conversion rate, gross margin, optimization, threshold value, selection strategy, reaction vessel, conversion rate, gross margin, optimization, threshold value, selection strategy, reaction vessel

**C. DOCUMENTS CONSIDERED TO BE RELEVANT**

| Category* | Citation of document, with indication, where appropriate, of the relevant passages | Relevant to claim No. |
| --- | --- | --- |
| A | CN 117280054 A (PT QMB NEW ENERGY MATERIALS et al.) 22 December 2023 (2023-12-22)<br>description, paragraphs 6-12 | 1-10 |
| A | AU 2011218742 A1 (VALE S.A.) 22 September 2011 (2011-09-22)<br>entire document | 1-10 |
| A | CA 618826 A (FREEPORT SULPHUR COMPANY) 25 April 1961 (1961-04-25)<br>entire document | 1-10 |
| A | CN 101338377 A (CENTRAL SOUTH UNIVERSITY) 07 January 2009 (2009-01-07)<br>entire document | 1-10 |
| A | CN 117295833 A (PT QMB NEW ENERGY MATERIALS et al.) 26 December 2023 (2023-12-26)<br>entire document | 1-10 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **26 May 2025** | **04 June 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2024/122779**

| Patent document cited in search report | | | Publication date (day/month/year) | Patent family member(s) | | | Publication date (day/month/year) |
|---|---|---|---|---|---|---|---|
| CN | 117280054 | A | 22 December 2023 | None | | | |
| AU | 2011218742 | A1 | 22 September 2011 | AU | 2011218742 | B2 | 23 January 2014 |
| CA | 618826 | A | 25 April 1961 | None | | | |
| CN | 101338377 | A | 07 January 2009 | None | | | |
| CN | 117295833 | A | 26 December 2023 | None | | | |

Form PCT/ISA/210 (patent family annex) (July 2022)